# EUROPEAN PATENT APPLICATION

(11) **EP 3 381 396 A1**
(43) Date of publication of application: **03.10.2018**
(21) Application number: 18164943.5
(22) Date of filing: 29.03.2018
(51) Int. Cl.: A61B 18/14, A61B 18/16, A61B 5/06

(54) **BALLOON CATHETER WITH LARGE AREA ELECTRODES**

(30) Priority: 31.03.2017 US 201715476191
(71) Applicant: Biosense Webster (Israel) Ltd., Yokneam 2066717 (IL)
(72) Inventor: GOVARI, Assaf, 2066717 Yokneam (IL); BEECKLER, Christopher Thomas, Irwindale, CA California 91706 (US)
(74) Representative: Carpmaels & Ransford LLP

(57) **Abstract**

A medical apparatus, including a probe having a distal end configured for insertion into a body cavity and containing a lumen that opens through the distal end, and an inflatable balloon deployable through the lumen into the body cavity such that when the balloon is deployed through the lumen and inflated, a distal pole on a distal side of the balloon is located opposite the lumen. In embodiments of the present invention, the medical apparatus also includes an electrode attached to the distal side of the inflatable balloon and extending over at least 50% of an area of the distal side of the balloon that is within 30° of arc from the distal pole.

## Description

### FIELD OF THE INVENTION

The present invention relates generally to invasive medical probes, and specifically to a balloon catheter comprising one or more large area electrodes.

### BACKGROUND OF THE INVENTION

A balloon catheter comprises an inflatable balloon at its distal end that can be inflated and deflated as necessary. In operation, the balloon is typically deflated while the catheter is inserted into a body cavity (e.g., a heart) of a patient, inflated in order to perform the necessary procedure, and deflated again upon completing the procedure.

U.S. Patent Application 2012/0310233 to Dimmer et al., whose disclosure is incorporated herein by reference, describes a balloon catheter comprising electrodes that can have different shapes and configurations. The electrodes are attached to an inflatable balloon having a diameter between five and twenty five millimeters.

U.S. Patent 8,295,902 to Salahieh et al., whose disclosure is incorporated herein by reference, describes a tissue electrode assembly that includes electrodes that can have different shapes, sizes and patterns. The configuration of the electrodes influences the amount and ablation lines of energy delivered by the electrodes to body tissue.

European Patent Application EP 2,923,666 to Govari et al., whose disclosure is incorporated herein by reference, describes a catheter with a balloon having one or more ablation electrodes and multiple microelectrodes that are configured to measure temperature. In some embodiments, the microelectrodes are disposed circumferentially about a longitudinal axis of an exterior wall of the balloon. In alternative embodiments, the microelectrodes are disposed on a flexible circuit substrate and adhered to the exterior wall of the balloon.

International Patent Application WO 2014/070999 to Toth et al., whose disclosure is incorporated herein by reference, describes a probe that can be used for micro-ablation procedures and includes a plurality of electrodes. The electrodes may comprise microelectrodes, stimulation electrodes, and ablation electrodes.

United States Patent Application 2009/0076498 to Vahid et al., whose disclosure is incorporated herein by reference, describes a visualization and ablation system that includes a visualization balloon having ablation electrodes on a distal front surface of the balloon.

United States Patent Application 2008/0319350 to Wallace et al., whose disclosure is incorporated herein by reference, describes a device configured to measure a size of a body lumen and to ablate tissue that uses the measurement to normalize delivery of ablation energy from a balloon to a luminal target of varying circumference. The ablation energy is delivered by an energy delivery element such as a radio-frequency electrode, an array of electrodes, or solid-state circuitry that can be arranged directly on the expandable balloon, or arranged on an electrode support that is itself engaged around the balloon.

United States Patent 6,771,996 to Bowe et al., whose disclosure is incorporated herein by reference, describes an ablation and high-resolution mapping catheter system for pulmonary vein foci elimination. The catheter system may include an inflatable balloon having a plurality of mapping electrodes arranged in an array at the distal end of the balloon.

Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

The description above is presented as a general overview of related art in this field and should not be construed as an admission that any of the information it contains constitutes prior art against the present patent application.

### SUMMARY OF THE INVENTION

There is provided, in accordance with an embodiment of the present invention, a medical apparatus, including a probe having a distal end configured for insertion into a body cavity and containing a lumen that opens through the distal end, an inflatable balloon deployable through the lumen into the body cavity such that when the balloon is deployed through the lumen and inflated, a distal pole on a distal side of the balloon is located opposite the lumen, and an electrode attached to the distal side of the inflatable balloon and extending over at least 50% of an area of the distal side of the balloon that is within 30° of arc from the distal pole.

In some embodiments, the electrode has a non-polygonal shape. In additional embodiments, the electrode is symmetrical around the distal pole. In further embodiments, the balloon has a non-elongated spherical shape when inflated.

In some embodiments, the distal side of the balloon has a continuously smooth surface. In additional embodiments where the distal side of the balloon has a continuously smooth surface, the electrode may include multiple sub-electrodes. In further embodiments where the distal side of the balloon has a continuously smooth surface, the electrode may include a first electrode that does not cover the distal pole and having a first size, and the medical apparatus may include a second electrode having a second size smaller than the first size and attached to the distal pole of the inflatable balloon.

In supplemental embodiments, the medical apparatus may include additional electrodes attached to the distal side of the inflatable balloon. In some embodiments, the balloon is not inflated when deployed through the lumen, the balloon has a balloon diameter less than or equal to eight millimeters when inflated, and the lumen has a lumen diameter of 2.5 millimeters. In additional embodiments, the electrode extending over at least 50% of an area of the distal side of the balloon that is within 30° of arc from the distal pole includes the electrode extending over at least 75% of the area of the distal side of the balloon that is within 30° of arc from the distal pole.

There is also provided, in accordance with an embodiment of the present invention, a method, including providing a probe having a distal end configured for insertion into a body cavity and containing a lumen that opens through the distal end, providing an inflatable balloon that is deployable through the lumen into the body cavity such that when the balloon is deployed through the lumen and inflated, a distal pole on a distal side of the balloon is located opposite the lumen, and attaching, to the distal side of the inflatable balloon, an electrode that extends over at least 50% of an area of the distal side of the balloon that is within 30° of arc from the distal pole.

There is additionally provided, in accordance with an embodiment of the present invention, a method, including inserting a distal end of a medical probe into a body cavity of a patient, the medical probe including a lumen that opens through the distal end, an inflatable balloon deployable through the lumen into the body cavity such that when the balloon is deployed through the lumen and inflated, a distal pole on a distal side of the balloon is located opposite the lumen, and an electrode attached to the distal side of the inflatable balloon and extending over at least 50% of an area of the distal side of the balloon that is within 30° of arc from the distal pole. The method also includes selecting, in the body cavity, an area of tissue to ablate in a region distal to the catheter, controlling an inflation pressure of the balloon responsively to a size of the area, pressing the distal side of the balloon against the selected area of the tissue, and ablating the selected area of the tissue.

In some embodiments, controlling the inflation pressure includes controlling an irrigation flow rate.

### BRIEF DESCRIPTION OF THE DRAWINGS

The disclosure is herein described, by way of example only, with reference to the accompanying drawings, wherein:
Figure 1 is a schematic, pictorial illustration of a medical system comprising a medical probe whose distal end comprises a balloon, in accordance with an embodiment of the present invention;
Figure 2 is a schematic pictorial illustration of the distal end comprising multiple ablation electrodes and multiple microelectrodes attached to the balloon, in accordance with an embodiment of the present invention;
Figure 3 is a schematic cutaway view of the distal end of the medical probe, in accordance with an embodiment of the present invention;
Figure 4 is a flow diagram that schematically illustrates a method of using the medical probe to perform an ablation procedure on endocardial tissue, in accordance with an embodiment of the present invention;
Figure 5 is a schematic detail view of the distal end of the medical probe performing the ablation procedure on the endocardial tissue while the balloon is fully inflated, in accordance with an embodiment of the present invention; and
Figure 6 is a schematic detail view of the distal end of the medical probe performing the ablation procedure on the endocardial tissue while the balloon is partially inflated, in accordance with an embodiment of the present invention.

### DETAILED DESCRIPTION OF EMBODIMENTS

In embodiments of the present invention, a medical probe, in this case a balloon catheter, comprises a distal end configured for insertion into a body cavity, and the probe contains a lumen that opens through the distal end. The medical probe also comprises an inflatable balloon that is deployable through the lumen into the body cavity such that when the balloon is deployed through the lumen and inflated, a distal pole on a distal side of the balloon is located opposite the lumen. The medical probe further comprises an electrode that is attached to the distal side of the inflatable balloon and extends over at least 50% of an area of the distal side of the balloon that is within 30° of arc from the distal pole.

Balloon catheters implementing embodiments of the present invention enable ablation of tissue in contact with the distal pole of the balloon. In embodiments of the present invention, varying the inflation pressure in the balloon impacts the compliance and size of the balloon, which therefore impacts the percentage of surface area of the electrode that is in contact with tissue in a body cavity. Therefore, as described hereinbelow, during a cardiac ablation procedure, a medical professional can control the size of the ablation area by controlling the inflation pressure in the balloon.

### SYSTEM DESCRIPTION

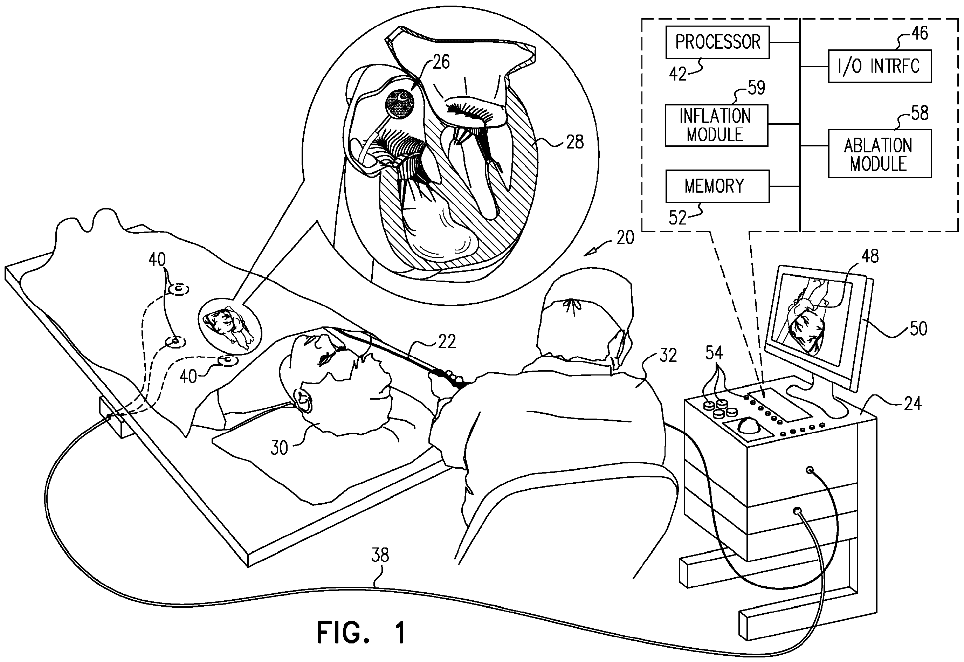

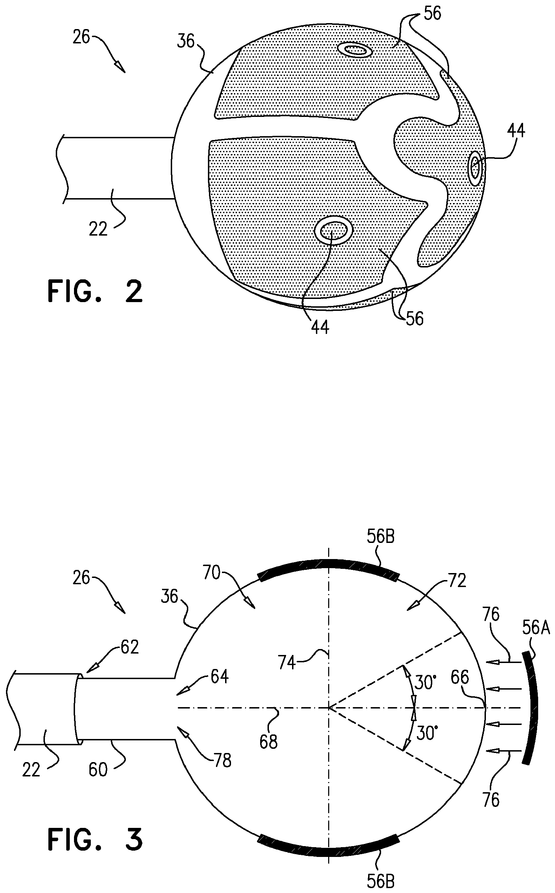

Figure 1 is a schematic, pictorial illustration of a medical system 20 comprising a medical probe 22 and a control console 24, and Figure 2 is a schematic pictorial illustration of a distal end 26 of the medical probe, in accordance with an embodiment of the present invention. Medical system 20 may be based, for example, on the CARTO® system, produced by Biosense Webster Inc. (Diamond Bar, California, U.S.A.). In embodiments described hereinbelow, medical probe 22 comprises a balloon catheter that is used for diagnostic or therapeutic treatment, such as for performing ablation procedures in a heart 28 of a patient 30. Alternatively, medical probe 22 may be used, *mutatis mutandis,* for other therapeutic and/or diagnostic purposes in the heart or in other body organs.

During a medical procedure, a medical professional 32 inserts medical probe 22 into a biocompatible sheath (not shown) that has been pre-positioned in a body cavity (e.g., a chamber of heart 28) of the patient so that an inflatable balloon 36 (Figure 2) affixed to distal end 26 of the medical probe enters the body cavity. Balloon 36 is typically formed from bio-compatible material such as polyethylene terephthalate (PET), polyurethane, Nylon, or Pebax.

Control console 24 is connected, by a cable 38, to body surface electrodes, which typically comprise adhesive skin patches 40 that are affixed to patient 30. Control console 24 comprises a processor 42 that determines position coordinates of distal end 26 inside heart 28 based on impedances measured between adhesive skin patches 40 and one or more electrodes 44 (also referred to herein as microelectrodes 44) that are attached to an exterior wall of balloon 36. While embodiments herein describe using microelectrodes 44 as location sensors, using the microelectrodes to perform other tasks (e.g., measuring electrical activity of heart 28) during a medical procedure is considered to be within the spirit and scope of the present invention.

Processor 42 typically comprises a general-purpose computer, with suitable front end and interface circuits for receiving signals from elements of medical probe 22 (e.g., microelectrodes 44) and controlling the other components of control console 24. Processor 42 may be programmed in software to carry out the functions that are described herein. The software may be downloaded to control console 24 in electronic form, over a network, for example, or it may be provided on non-transitory tangible media, such as optical, magnetic or electronic memory media. Alternatively, some or all of the functions of processor 42 may be carried out by dedicated or programmable digital hardware components.

Although the medical system shown in Figures 1 and 2 uses impedance-based sensing to measure a location of distal end 26, other position tracking techniques may be used (e.g., techniques using magnetic-based sensors). Impedance-based position tracking techniques are described, for example, in U.S. Patents 5,983,126, 6,456,864 and 5,944,022, whose disclosures are incorporated herein by reference. Magnetic position tracking techniques are described, for example, in U.S. Patents 5,391,199, 5,443,489, 6,788,967, 6,690,963, 5,558,091, 6,172,499 6,177,792, whose disclosures are incorporated herein by reference. The methods of position sensing described hereinabove are implemented in the above-mentioned CARTO® system and are described in detail in the patents cited above.

Control console 24 also comprises an input/output (I/O) communications interface 46 that enables the control console to transfer signals from, and/or transfer signals to electrodes 44 in medical probe 22 and adhesive skin patches 40. Based on signals received from microelectrodes 44 and adhesive skin patches 40, processor 42 can generate a map 48 that shows the position of balloon 36 in the patient's body. During the procedure, processor 42 can present map 48 to medical professional 32 on a display 50, and store data representing the map in a memory 52. Memory 52 may comprise any suitable volatile and/or non-volatile memory, such as random access memory or a hard disk drive. In some embodiments, medical professional 32 can manipulate map 48 using one or more input devices 54. In alternative embodiments, display 50 may comprise a touchscreen that can be configured to accept inputs from medical professional 32, in addition to presenting image 48.

In embodiments of the present invention, distal end 26 comprises one or more electrodes 56, that are typically used for ablation and so are also referred to herein as ablation electrodes 56, attached to the exterior wall of balloon 36. In the configuration shown in Figure 2, ablation electrodes 56 have non-polygonal shapes, and microelectrodes 44 are positioned in "islands" within the ablation electrodes. Electrodes 44 and 56 can be fabricated with the balloon and typically comprise gold overlaying the exterior wall of balloon 36.

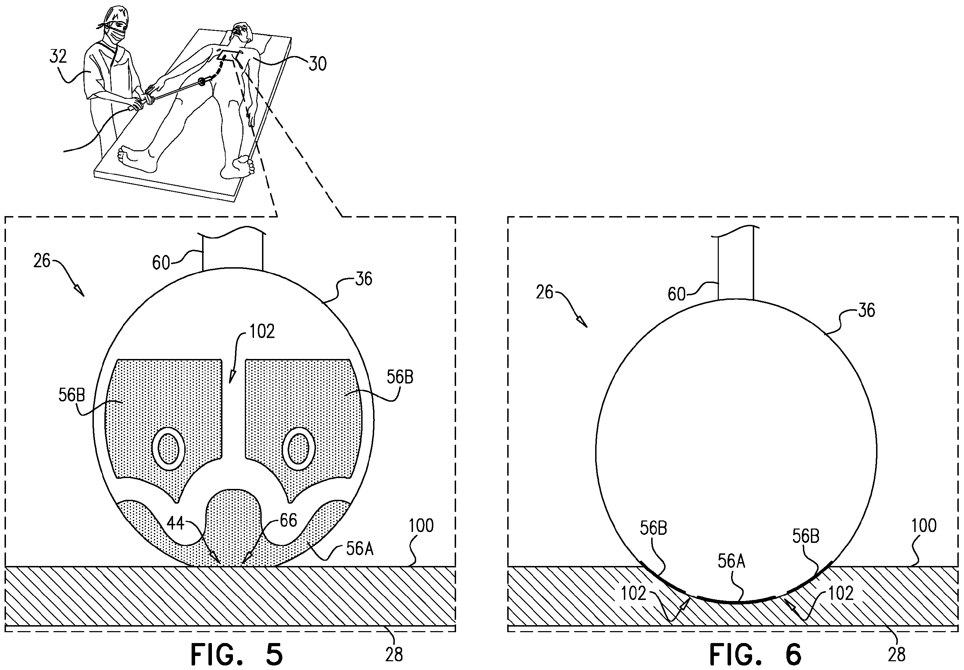

Control console 24 also comprises an ablation module 58, and an inflation module 59. Ablation module 58 is configured to monitor and control ablation parameters such as the level and the duration of ablation power (e.g., radio-frequency energy) conveyed to ablation electrodes 56. Inflation module 59 is configured to monitor and control the inflation pressure in balloon 36. As described in the description referencing Figures 5 and 6 hereinbelow, medical professional 32 can control the inflation pressure in the balloon in order to adjust the amount of cardiac tissue that is in contact with electrode(s) 56 during an ablation procedure.

In some embodiments, inflation module 59 can use irrigation fluid to inflate balloon 36, and control the inflation pressure in the balloon by controlling a flow rate of the irrigation fluid into the balloon. In these embodiments balloon 36 comprises multiple small fenestrations (not shown) that allow the irrigation fluid to exit the balloon. These fenestrations are typically 0.025-0.500 millimeters (mm) (e.g., 0.089mm) in diameter.

Figure 3 is a schematic cutaway view of distal end 26, in accordance with an embodiment of the present invention. Balloon 36 is affixed to a tubular shaft 60 that is configured to extend from a distal end of a lumen 62 of medical probe 22, and the balloon is configured to be deployed through the lumen into a body cavity such as heart 28.

Balloon 36 comprises a proximal pole region 64 and a distal pole 66 that lie on a longitudinal equator 68 of the balloon. Balloon 36 also comprises a proximal side 70 and a distal side 72 that are separated by a latitudinal equator 74 of the balloon. Proximal side 70 comprises a first hemisphere terminated by latitudinal equator 74 that includes proximal pole region 64, and distal side 72 comprises a second hemisphere terminated by the latitudinal equator that includes distal pole 66.

In embodiments of the present invention, balloon 36 does not comprise any structural elements (e.g., an extender shaft) within the balloon. Additionally, balloon 36 comprises a main aperture 78 at proximal pole region 64 that connects the balloon to tubular shaft 60 (i.e., for inflation/deflation). In other words, unlike other balloons used for ablation known in the art, balloon 36 does not comprise a through extender shaft.

Furthermore, distal side 72 has a continuously smooth surface (i.e., there are no large apertures on the distal side) and balloon 36 has a non-elongated spherical shape when inflated. In some embodiments, medical probe 22 has a diameter of 2.5 millimeters, and balloon 36 can have a diameter of up to eight millimeters when inflated. In a non-inflated state, balloon 36 can be retracted into the medical probe. The inventors have found that using these dimensions, the balloon can form its non-elongated spherical shape when inflated, yet retract to fit within probe 22 when deflated.

Ablation electrodes 56 are differentiated herein by appending a letter to the identifying numeral, so that the ablation electrodes comprise ablation electrodes 56A and 56B. In embodiments of the present invention, ablation electrode 56A is attached to balloon 36 (i.e., as indicated by arrows 76) so that ablation electrode 56A extends over at least 50% of an area on distal side 72 that is within 30° of arc from distal pole 66. In some embodiments, ablation electrode 56A can extend over at least 75% of the area on distal side 72 that is within 30° of arc from distal pole 66. In additional embodiments, the shape of ablation electrode 56A is symmetrical around distal pole 66. In the example shown in Figure 3, ablation electrodes 56B are attached to balloon 36 and encompass both proximal side 70 and distal side 72.

In further embodiments, as shown in Figure 3, distal end 72 may not include any microelectrodes 44. In these embodiments, electrodes 56 can be used as position sensors (i.e., in addition to being used to ablate tissue). In additional embodiments, ablation electrode 56A may comprise multiple "sub-electrodes" that extend over at least 50% of an area on distal side 72 that is within 30° of arc from distal pole 66. For simplicity, connections of electrodes 56 and microelectrodes 44 to interface 46 and module 58 are not shown. In some embodiments, the connections are made by wires (not shown) running from the inside of the balloon to the outer surface of the balloon. The electrical connections can be formed with conductive epoxy or welding.

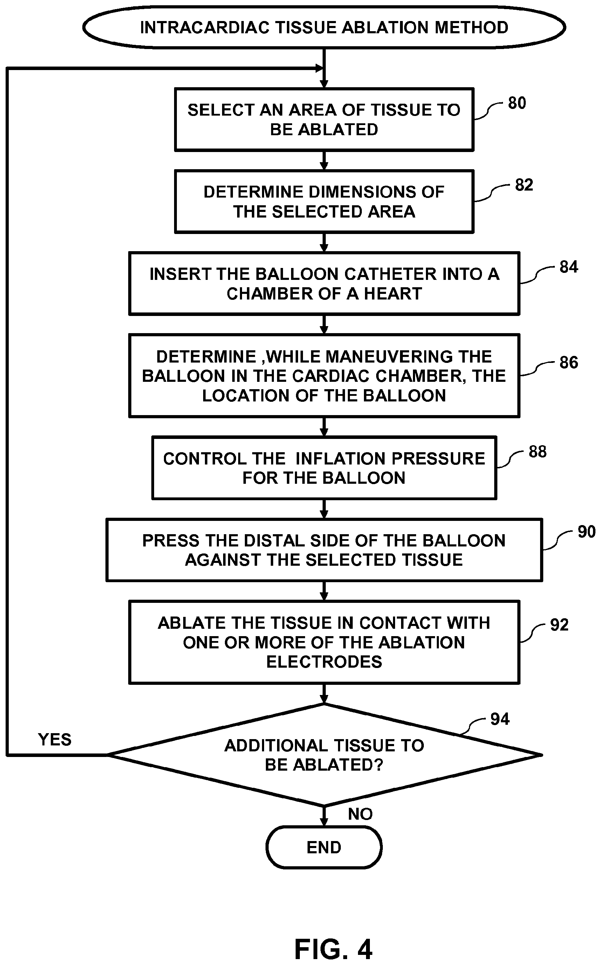

Figure 4 is a flow diagram that schematically illustrates a method of using medical probe 22 to perform an ablation procedure, and Figures 5 and 6 (drawn to different scales) are schematic detail views of distal end 26 while the medical probe performs the ablation procedure on tissue in heart 28, in accordance with an embodiment of the present invention. Figure 5 illustrates distal end when balloon 36 is under low pressure (0.5-5.0 P.S.I.), and Figure 6 illustrates the distal end when the balloon is under high pressure. When under high pressure (e.g., >5.0 P.S.I.) the diameter of the balloon increases. This increase comes preferentially from enlargement of gaps 102 between electrodes 56. When enlarged, more electrode surface comes into contact with the tissue. This allows for the creation of a larger and deeper lesion.

Under low pressure, balloon 36 inflates to a first diameter (e.g., 2.5mm), and under high pressure, the balloon inflates to a second diameter (e.g., 8.0mm) that is greater than the first diameter. As shown in Figure 5, while pressing distal side 72 against tissue 100 during an ablation procedure, only a small portion of electrode 56A is in contact with the tissue when balloon 36 is inflated to the first diameter. However, as shown in Figure 6, while pressing distal side 72 against tissue 100 during an ablation procedure, almost all (or all) of electrodes 56A and 56B are in contact with the tissue when balloon 36 is inflated to the second diameter. In balloon catheters implementing embodiments of the present invention, inflating balloon 36 using higher pressure stretches the elastic biocompatible material in the balloon (i.e., electrodes 56 typically do not stretch), thereby increasing the surface area of gaps 102, and "pushing" electrodes 56B forward (i.e., towards tissue 100).

In a selection step 80, medical professional 32 selects, e.g., from a previously generated map of heart 28, an area of tissue 100 in heart 28 that is in a region of the tissue distal to medical probe 22, and in a first determination step 82, processor 42 determines dimensions (and therefore a size) of the selected area. In an insertion step 84, medical professional 32 manipulates medical probe 22 so that distal end 26 enters a chamber of heart 28, and while maneuvering the medical probe in the cardiac chamber, processor 42 determines, based on impedances measured by microelectrodes 44, a current location of balloon 36 in a second determination step 86.

In a control step 88, in response to the determined size of the selected area, medical professional 32 can control, using inflation module 59, the inflation pressure (and therefore the size) of balloon 36. In some embodiments, as shown in Figure 6, one or more electrodes 56 conform to and cover the selected area in response to the exerted force. In some embodiments, upon processor 42 determining the size of the selected area, the processor can determine the inflation pressure responsively to the size of the area (i.e., in order to ensure that one or more ablation electrodes cover the selected area when distal side 72 of balloon 36 presses against tissue 100).

In a first positioning step 90, medical professional 32 positions medical probe 22 so that distal side 72 of balloon 36 presses against the selected area of tissue 100, and in an ablation step 92, one or more ablation electrodes 56 deliver ablation energy received from ablation module 58 to the tissue in order to perform the ablation.

In embodiments of the present invention, the inflation pressure in balloon 36 influences the compliancy of the balloon. Therefore, the number of ablation electrodes 56 used, and the size of the area being ablated by the ablation electrodes, are dependent on the inflation pressure in the balloon. In the example shown in Figure 6, balloon 36 is inflated using a high inflation pressure thereby expanding the balloon and placing more electrodes into contact with the tissue. In Figure 5, the balloon is inflated using a low inflation pressure, thereby making the balloon smaller and presenting less electrodes towards the distal face of the balloon (i.e., less electrodes make contact with the tissue).

As shown in Figure 5, only a portion of ablation electrode 56A is in contact (and can therefore ablate) with the tissue during the ablation procedure when balloon 36 is inflated using a low inflation pressure. In embodiments where balloon 36 comprises a given microelectrode 44 that encompasses distal pole 66 (i.e., as shown in Figure 2), heat generated in response to ablation energy delivered by ablation electrode 56A (i.e., that surrounds the given microelectrode) can conduct to nearby tissue (and therefore ablate) the tissue that is in contact with the given microelectrode (i.e., the portion the exterior wall of balloon 36 surrounding distal pole 66 that is not covered by ablation electrode 56A).

As shown in Figure 6, the increased size of balloon 36 enables almost all of ablation electrode 56A and portions of ablation electrodes 56B to be in contact with tissue 100. In other words, the area of ablation electrodes 56 that can deliver radio-frequency energy to tissue 100 for ablation is related to the inflation pressure in balloon 36, so that as the pressure increases, the area increases (and *vice versa*)*.* Additionally, in the high pressure state, while distal side 72 is pressed against the tissue, there is less interference from surrounding blood, so that ablation electrodes 56 can create deeper lesions and ablate larger areas of tissue.

While ablation electrodes 56 are in contact with tissue 100, there are gaps (i.e., open space) 102 on balloon 36 that is not covered by the ablation electrodes. In operation, heat in the tissue that is generated in response to ablation energy delivered by ablation electrodes 56 (i.e., that surround the open space) can conduct to nearby tissue (and therefore ablate) the tissue that is in contact with any gaps 102 that are in contact with tissue 100.

Returning to the flow diagram, in a comparison step 94, if there is any additional tissue that needs to be ablated, then the method continues with step 80. Returning to step 96, if there is no further tissue 100 that needs to be ablated, then the ablation procedure is complete and the method ends.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

### ASPECTS OF THE INVENTION

1. A method, comprising:
   inserting a distal end of a medical probe into a body cavity of a patient, the medical probe comprising:
   a lumen that opens through the distal end,
   an inflatable balloon deployable through the lumen into the body cavity such that when the balloon is deployed through the lumen and inflated, a distal pole on a distal side of the balloon is located opposite the lumen, and
   an electrode attached to the distal side of the inflatable balloon and extending over at least 50% of an area of the distal side of the balloon that is within 30° of arc from the distal pole;
   selecting, in the body cavity, an area of tissue to ablate in a region distal to the catheter;
   controlling an inflation pressure of the balloon responsively to a size of the area;
   pressing the distal side of the balloon against the selected area of the tissue; and
   ablating the selected area of the tissue.
2. The method according to aspect 1, wherein controlling the inflation pressure comprises controlling an irrigation flow rate.

## Claims

1. A medical apparatus, comprising:
a probe having a distal end configured for insertion into a body cavity and containing a lumen that opens through the distal end;
an inflatable balloon deployable through the lumen into the body cavity such that when the balloon is deployed through the lumen and inflated, a distal pole on a distal side of the balloon is located opposite the lumen; and
an electrode attached to the distal side of the inflatable balloon and extending over at least 50% of an area of the distal side of the balloon that is within 30° of arc from the distal pole.

2. The medical apparatus according to claim 1, and comprising additional electrodes attached to the distal side of the inflatable balloon.

3. The medical apparatus according to claim 1, wherein the balloon is not inflated when deployed through the lumen, wherein the balloon has a balloon diameter less than or equal to eight millimeters when inflated, and wherein the lumen has a lumen diameter of 2.5 millimeters.

4. The medical apparatus according to claim 1, wherein the electrode extending over at least 50% of an area of the distal side of the balloon that is within 30° of arc from the distal pole comprises the electrode extending over at least 75% of the area of the distal side of the balloon that is within 30° of arc from the distal pole.

5. A method, comprising:
providing a probe having a distal end configured for insertion into a body cavity and containing a lumen that opens through the distal end;
providing an inflatable balloon that is deployable through the lumen into the body cavity such that when the balloon is deployed through the lumen and inflated, a distal pole on a distal side of the balloon is located opposite the lumen; and
attaching, to the distal side of the inflatable balloon, an electrode that extends over at least 50% of an area of the distal side of the balloon that is within 30° of arc from the distal pole.

6. The medical apparatus according to claim 1 or the method according to claim 5, wherein the electrode has a non-polygonal shape.

7. The medical apparatus according to claim 1 or the method according to claim 5, wherein the electrode is symmetrical around the distal pole.

8. The medical apparatus according to claim 1 or the method according to claim 5, wherein the balloon has a non-elongated spherical shape when inflated.

9. The medical apparatus according to claim 1 or the method according to claim 5, wherein the distal side of the balloon has a continuously smooth surface.

10. The medical apparatus or the method according to claim 9, wherein the electrode comprises multiple sub-electrodes.

11. The medical apparatus according to claim 9, wherein the electrode comprises a first electrode that does not cover the distal pole and having a first size, and comprising a second electrode having a second size smaller than the first size and attached to the distal pole of the inflatable balloon.

12. The method according to claim 9, wherein the electrode comprises a first electrode that does not cover the distal pole and having a first size, and comprising attaching, to the distal pole of the inflatable balloon, a second electrode having a second size smaller than the first size.

13. The method according to claim 5, and comprising attaching additional electrodes to the distal side of the inflatable balloon.

14. The method according to claim 5, wherein the balloon is not inflated when deployed through the lumen, wherein the balloon has a balloon diameter less than or equal to eight millimeters when inflated, and wherein the lumen has a lumen diameter of 2.5 millimeters.

15. The method according to claim 14, wherein the electrode that extends over at least 50% of an area of the distal side of the balloon that is within 30° of arc from the distal pole comprises the electrode extending over at least 75% of the area of the distal side of the balloon that is within 30° of arc from the distal pole.
